# EUROPÄISCHE PATENTSCHRIFT

(11) **EP 4 203 849 B1**
(45) Veröffentlichungstag und Bekanntmachung des Hinweises auf die Patenterteilung: **02.10.2024**
(21) Anmeldenummer: 21766402.8
(22) Anmeldetag: 13.08.2021
(51) Int. Cl.: A61F 2/16

(54) **OPHTHALMOCHIRURGISCHER INJEKTOR**
OPHTHALMO-SURGICAL INJECTOR
INJECTEUR OPHTHALMO-CHIRURGICAL

(30) Priorität: 28.08.2020 DE 102020122597
(43) Veröffentlichungstag der Anmeldung: 05.07.2023
(73) Patentinhaber: Carl Zeiss Meditec AG, 07745 Jena (DE)
(72) Erfinder: GERLACH, Mario, 16548 Glienicke-Nordbahn (DE)
(74) Vertreter: PATERIS Patentanwälte PartmbB
(86) Internationale Anmeldenummer: PCT/EP2021/072638
(87) Internationale Veröffentlichungsnummer: WO 2022/043102

(56) Entgegenhaltungen:
- WO-A1-2020/151908
- US-A1- 2006 229 634
- US-A1- 2010 160 926

## Beschreibung

Die Erfindung betrifft einen ophthalmochirurgischen Injektor für ein Einführen einer Intraokularlinse in den Kapselsack eines Auges.

Bei einer Kataraktbehandlung eines Auges wird herkömmlich nur ein kleiner Schnitt in die Hornhaut des Auges eingebracht, der so groß ist, dass eine Spitze eines Injektors durch den Schnitt in das Auge eingeführt werden kann. Nachdem der Schnitt in die Hornhaut eingebracht wurde, wird die Linse des Auges üblicherweise mittels Phakoemulsifikation zerkleinert und anschließend aus dem Kapselsack des Auges abgesaugt. Danach wird eine Intraokularlinse mittels des Injektors in das Auge eingesetzt. Dabei verschiebt ein Stempel des Injektors die Intraokularlinse innerhalb des Injektors und aus dem Injektor heraus. Während die Intraokularlinse verschoben wird, wird die Intraokularlinse gefaltet, so dass sie durch die Spitze des Injektors und den Schnitt in der Hornhaut passt. Die Spitze wird durch den Schnitt in der Hornhaut in den Kapselsack eingeführt und die gefaltete Intraokularlinse wird mittels des Injektors durch die Spitze in den Kapselsack geschoben, wobei darauf die Intraokularlinse sich in dem Kapselsack entfaltet und somit die ursprüngliche Linse ersetzt.

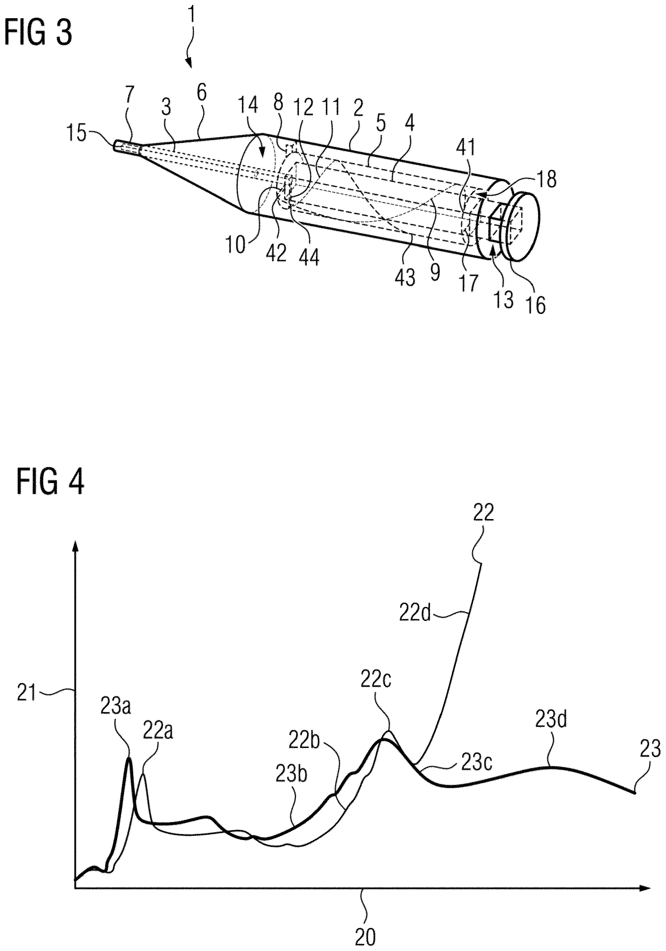

Bei dem Verschieben der Intraokularlinse durch verschiedene Abschnitte des Injektors treten unterschiedlich hohe Widerstandskräfte auf. In Figur 4 ist eine beispielhafte Kraft-Weg-Kurve 22 eines Stempels eines herkömmlichen Injektors dargestellt, wobei über die horizontale Achse der Verschiebeweg 20 des Stempels und über die vertikale Achse die bei dem Verschieben aufgewandte Kraft 21 aufgetragen ist. Es ist erkennbar, dass entlang den Verschiebeweges des Injektors stark verschiedene Kräfte aufgewendet werden müssen. Das lokale Maximum der Kraft mit Bezugszeichen 22a resultiert von einer Lösung des Stempels von einem Sicherheitsverschluss. Der Anstieg der Kraft in dem mit Bezugszeichen 22b bezeichneten Bereich resultiert daher, dass die Intraokularlinse von einer Lagerkammer in eine sich in Richtung zu der Spitze hin verjüngenden Faltkammer geschoben wird, in der die Intraokularlinse gefaltet und elastisch verformt wird. Der Abfall der Kraft in dem mit Bezugszeichen 22c bezeichneten Bereich ist dadurch zu erklären, dass die Intraokularlinse von der Faltkammer in die Spitze geschoben wird, wobei die Spitze sich mit einem flacheren Winkel in Richtung zu dem Ausgang der Spitze als die Faltkammer verjüngt. Der besonders steile Anstieg der Kraft im Bereich des Bezugszeichens 22d resultiert daher, dass die Intraokularlinse und eine Stempelspitze des Stempels am Ende der Spitze komprimiert werden. Die starke Kraft kann abrupt kollabieren, wenn die Intraokularlinse den Injektor verlässt, was dazu führen kann, dass die Intraokularlinse mit einer hohen Geschwindigkeit und daher unkontrolliert in den Kapselsack gelangt.

WO 2020/151908 A1 offenbart einen Injektor zum Injizieren einer intraokularen Linse, wobei der Injektor eine Geschwindigkeitsregelung hat. US 2010/0160926 A1 beschreibt ein Gerät zum Injizieren einer Intraokularlinse und Techniken zum Kompensieren von Variationen im Widerstand beim Injizieren. US 2006/0229634 A1 offenbart eine Vorrichtung und ein Verfahren zum Einsetzen einer Intraokularlinse in ein Auge.

Es ist daher die Aufgabe der Erfindung, eine Kraft-Weg-Kurve zu verbessern, die bei einem Verschieben einer Intraokularlinse in einem Injektor auftritt.

Der erfindungsgemäße ophthalmochirurgische Injektor weist einen Injektorkörper, eine Spitze, mittels welcher eine Intraokularlinse aus dem Injektor herausdrückbar ist, einen ersten Stempel, der längsverschiebbar an dem Injektorkörper gelagert ist und einen ersten Vorsprung aufweist, eine Welle, die rotierbar in dem Injektorkörper gelagert ist sowie eine erste Kulisse und eine zweite Kulisse aufweist, die jeweils um die Welle gewunden sind, und einen zweiten Stempel auf, der längsverschiebbar an dem Injektorkörper gelagert ist und einen zweiten Vorsprung aufweist, wobei der erste Vorsprung in die erste Kulisse eingreift, wodurch der erste Stempel eingerichtet ist, durch eine Längsverschiebung des ersten Stempels eine Rotation der Welle um eine Längsachse des Injektorkörpers zu bewirken, und der zweite Vorsprung in die zweite Kulisse eingreift, wodurch die Welle eingerichtet ist, durch die mittels des ersten Stempels bewirkte Rotation eine Längsverschiebung des zweiten Stempels zu bewirken.

Mittels der Welle ist eine Übersetzung zwischen der Längsverschiebung des ersten Stempels und der Längsverschiebung des zweiten Stempels geschaffen. Mittels der Steigung der ersten Kulisse und der Steigung der zweiten Kulisse kann ein Übersetzungsverhältnis der Übersetzung eingestellt werden. Das Übersetzungsverhältnis kann beispielsweise definiert werden als das Verhältnis aus einer an dem zweiten Stempel anliegenden Kraft zu einer an dem ersten Stempel anliegenden Kraft. Indem das Übersetzungsverhältnis eingestellt wird, kann eine Kraft-Weg-Kurve einer Kraft verbessert werden, die bei der Längsverschiebung des ersten Stempels aufzubringen ist. Beispielsweise ist es denkbar, das Übersetzungsverhältnis so zu wählen, dass an dem zweiten Stempel eine stärkere Kraft als an dem ersten Stempel anliegt. Dadurch ist von einem Operateur eine geringere Kraft auf den ersten Stempel auszuüben, als wenn die Übersetzung nicht vorgesehen wäre. Die Steigung der ersten Kulisse und die Steigung der zweiten Kulisse können beispielsweise definiert sein als die Neigung der jeweiligen Kulisse gegen die Umfangsrichtung der Welle. Damit die erste Kulisse und die zweite Kulisse gewunden sind und somit die Längsverschiebung des ersten Stempels zu der Rotation der Welle führt sowie die Rotation der Welle zu der Längsverschiebung des zweiten Stempels führt, sind die Steigungen der ersten Kulisse und der zweiten Kulisse verschieden von Null und von Unendlich.

Es ist auch denkbar, dass das Übersetzungsverhältnis während der Längsverschiebung des ersten Stempels und des zweiten Stempels variiert. Dadurch kann eine Kraft-Weg-Kurve während der Längsverschiebung des ersten Stempels und des zweiten Stempels so verändert werden, dass sie flacher wird, wodurch die Kraft-Weg-Kurve ebenfalls verbessert wird. Beispielweise ist es denkbar, in Bereichen der Längsverschiebung des zweiten Stempels, bei denen eine starke Kraft aufzuwenden ist, ein hohes Übersetzungsverhältnis zu wählen, und in Bereichen der Längsverschiebung des zweiten Stempels, in denen eine schwache Kraft aufzuwenden ist, ein niedriges Übersetzungsverhältnis zu wählen. Damit das Übersetzungsverhältnis variiert, ist es denkbar, dass die Steigung zumindest einer der beiden Kulissen bei der Längsverschiebung variiert, wobei es auch denkbar ist, dass bei der Längsverschiebung die Steigungen der beiden Kulissen variieren.

Es ist bevorzugt, dass der Injektor eingerichtet ist, die Längsverschiebung des ersten Stempels hin zu der Spitze in die Längsverschiebung des zweiten Stempels hin zu der Spitze zu übersetzen. Der zweite Stempel ist bevorzugt eingerichtet, durch seine Längsverschiebung die Intraokularlinse in die Spitze zu drücken und anschließend aus dem Injektor heraus zu drücken. Es ist zudem bevorzugt, dass die Längsverschiebung des ersten Stempels und des zweiten Stempels in einer Richtung erfolgt, die im Wesentlichen parallel zu der Axialrichtung der Welle ist.

Ein Steigungsverhältnis aus der Steigung der ersten Kulisse an der Position, in der der erste Vorsprung eingreift, zu der Steigung der zweiten Kulisse an der Position, in der der zweite Vorsprung eingreift, variiert bevorzugt bei der Längsverschiebung des ersten Stempels und des zweiten Stempels. Die Steigung der ersten Kulisse und die Steigung der zweiten Kulisse können beispielsweise definiert sein, als die Neigung der jeweiligen Kulisse gegen die Umfangsrichtung der Welle. Eine größere Steigung geht somit einher mit einer kürzeren Rotation der Welle pro Einheit der Längsverschiebung.

Mit dieser Definition ist das Steigungsverhältnis identisch mit dem Übersetzungsverhältnis und mit einem Kraftverhältnis einer an dem zweiten Stempel in der Axialrichtung der Welle angreifenden Kraft zu einer an dem ersten Stempel in der Axialrichtung der Welle angreifenden Kraft.

Es ist bevorzugt, dass das Steigungsverhältnis zumindest abschnittsweise bei der Längsverschiebung des ersten Stempels und des zweiten Stempels hin zu der Spitze größer wird. Dadurch ist es möglich, einen durch ein Falten und/oder ein elastisches Verformen der Intraokularlinse verursachten Anstieg der Kraft-Weg-Kurve abzuflachen. Das Steigungsverhältnis steigt besonders bevorzugt entlang eines Endbereichs eines Verschiebewegs des zweiten Stempels hin zu der Spitze monoton an. In dem Endbereich des Verschiebewegs des zweiten Stempels werden die Intraokularlinse und eine Stempelspitze des zweiten Stempels besonders stark komprimiert, was zu einem besonders starken Anstieg der Kraft-Weg-Kurve führt. Daher ist es besonders relevant, die Kraft-Weg-Kurve in diesem Bereich abzuflachen. Es ist auch denkbar, dass das Steigungsverhältnis streng monoton ansteigt. Es ist zudem besonders bevorzugt, dass das Steigungsverhältnis entlang eines Endbereichs eines Verschiebewegs des zweiten Stempels hin zu der Spitze größer als eins ist. Dadurch kann die von dem Operateur auf den ersten Stempel aufzuwendende Kraft verringert werden. Weil die Kraft, die aufzuwenden ist, um die Intraokularlinse in diesem Bereich längszuverschieben, besonders hoch ist, ist es besonders vorteilhaft, die von dem Operateur aufzuwendende Kraft in diesem Bereich zu verringern. Zudem kann damit vermieden werden, dass die Intraokularlinse mit einer hohen Geschwindigkeit und damit unkontrolliert den Injektor verlässt. Beispielsweise kann das Steigungsverhältnis an dem Ende der Kraft-Weg-Kurve größer als 3,0 und insbesondere größer als 4,0 sein.

Es ist bevorzugt, dass das Steigungsverhältnis entlang eines Anfangsbereichs eines Verschiebewegs des zweiten Stempels hin zu der Spitze größer als eins ist. Dadurch ist es beispielsweise möglich, einen durch ein Lösen eines Sicherheitsverschlusses durch den ersten Stempel verursachten Anstieg der Kraft-Weg-Kurve abzuflachen.

Die erste Kulisse weist bevorzugt einen ersten Abschnitt und einen zweiten Abschnitt, die jeweils um die Welle gewunden sind, sowie einen sich in der Axialrichtung der Welle orientierten Abschnitt auf, via den der erste Vorsprung durch seine Längsverschiebung weg von der Spitze von dem ersten Abschnitt in den zweiten Abschnitt gelangen kann. Dadurch ist es erforderlich, den ersten Stempel zweimal zu drücken, damit der erste Vorsprung die vollständige erste Kulisse abfährt. Dadurch kann ein besonders hohes Übersetzungsverhältnis realisiert werden. Es ist auch denkbar, dass mehr als zwei der Abschnitte, die um die Welle gewunden sind, und für jeweils für zwei benachbarte der gewundenen Abschnitte jeweils ein in der Axialrichtung der Welle orientierter Abschnitt vorgesehen sind, via den der erste Vorsprung durch seine Längsverschiebung weg von der Spitze von einem der benachbarten gewunden Abschnitte in den anderen der benachbarten gewundenen Abschnitte gelangen kann. Es ist besonders bevorzugt, dass der Injektor ein Vorspannmittel aufweist, das eingerichtet ist, den ersten Stempel in einer Richtung weg von der Spitze vorzuspannen. Dadurch gelangt der erste Vorsprung selbständig von dem ersten Abschnitt in den zweiten Abschnitt, sobald der Vorsprung an dem Ende des ersten Abschnitts angeordnet ist und die Kraft von dem ersten Stempel genommen wird. Bei dem Vorspannmittel kann es sich beispielsweise um eine Druckfeder handeln.

Es ist bevorzugt, dass der Injektor einen Anschlag aufweist, der eingerichtet ist, eine Bewegung der Welle hin zu der Spitze zu begrenzen.

Der Injektor weist bevorzugt eine erste Verdrehsicherung auf, die eingerichtet ist, eine Rotation des ersten Stempels relativ zu dem Injektorkörper zu unterbinden. Alternativ oder zusätzlich weist der Injektor bevorzugt eine zweite Verdrehsicherung auf, die eingerichtet ist, eine Rotation des zweiten Stempels relativ zu dem Injektorkörper zu unterbinden. Dadurch kann unterbunden werden, dass der erste Stempel und/oder der zweite Stempel bei ihrer Längsverschiebung in eine Rotation versetzt werden.

Es ist bevorzugt, dass die Welle eine Hohlwelle ist und somit einen Hohlraum aufweist, wobei der erste Stempel bei seiner Längsverschiebung zumindest teilweise und zumindest zeitweise in dem Hohlraum angeordnet ist und/oder wobei der zweite Stempel bei seiner Längsverschiebung zumindest teilweise und zumindest zeitweise in dem Hohlraum angeordnet ist. Dadurch kann der Injektor besonders platzsparend ausgeführt werden.

Es ist bevorzugt, dass die Welle eine Hohlwelle ist und somit einen Hohlraum aufweist, wobei die erste Kulisse und/oder die zweite Kulisse sich in einer Radialrichtung der Welle vollständig durch eine den Hohlraum begrenzenden Wand der Welle erstrecken. Alternativ ist es denkbar, dass bei der Hohlwelle die erste Kulisse und/oder die zweite Kulisse jeweils als eine Nut ausgebildet sind.

Alternativ ist bevorzugt, dass die Welle ohne einen Hohlraum in Ihrem Inneren ausgeführt ist und dass die erste Kulisse als eine Nut ausgebildet ist und/oder die zweite Kulisse als eine Nut ausgebildet ist.

Der Injektor weist bevorzugt die Intraokularlinse auf. Die Intraokularlinse ist bevorzugt in dem Injektorkörper angeordnet. Insbesondere kann die Intraokularlinse zu Beginn der Längsverschiebung des ersten Stempels, wobei zu dem Beginn der erste Stempel so weit wie möglich von der Spitze entfernt ist, in einer Lagerkammer des Injektors angeordnet sein, wobei die Lagerkammer an einer der Spitze abgewandten Seite der Faltkammer angeordnet ist.

Im Folgenden wird anhand der beigefügten schematischen Zeichnungen die Erfindung näher erläutert.
Figur 1 zeigt einen erfindungsgemäßen Injektor zu einem ersten Zeitpunkt.
Figur 2 zeigt den Injektor zu einem zweiten Zeitpunkt.
Figur 3 zeigt den Injektor zu einem dritten Zeitpunkt.
Figur 4 zeigt jeweils eine Kraft-Weg-Kurve für einen herkömmlichen Injektor und für den erfindungsgemäßen Injektor.
Figur 5 zeigt für eine erste beispielhafte Ausführungsform des Injektors eine Auftragung eines Verschiebewegs eines ersten Stempels des Injektors gegen einen zweiten Stempel des Injektors.
Figur 6 zeigt für die erste Ausführungsform jeweils eine Form einer ersten Kulisse und einer zweiten Kulisse.
Figur 7 zeigt für die erste Ausführungsform die Steigung der ersten Kulisse und die Steigung der zweiten Kulisse.
Figur 8 zeigt für die erste Ausführungsform eine Auftragung eines Steigungsverhältnisses aus der Steigung der ersten Kulisse zu der Steigung der zweiten Kulisse.
Figur 9 zeigt ein Steigungsverhältnis für eine zweite beispielhafte Ausführungsform des Injektors.

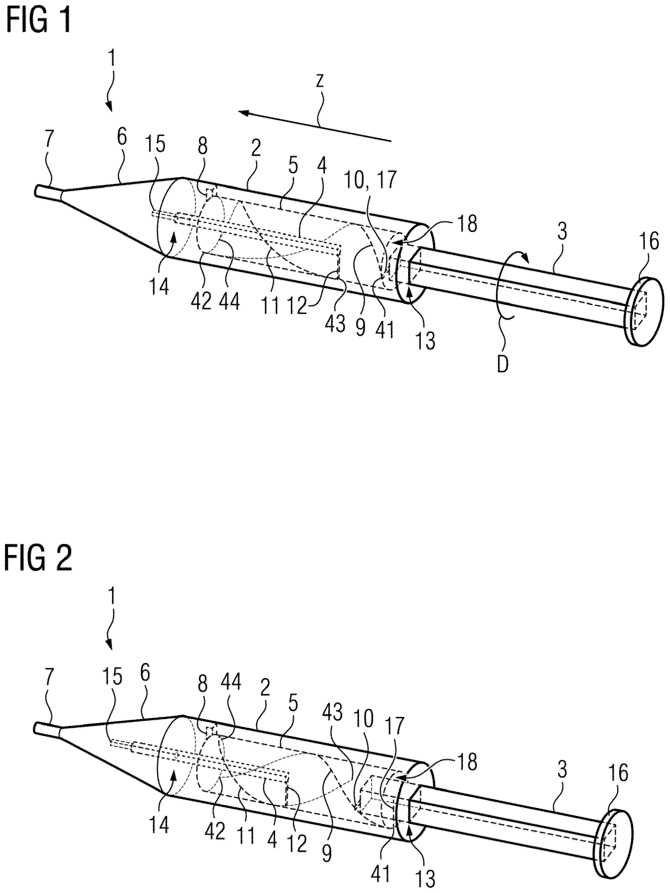

Wie es aus Figuren 1 bis 3 ersichtlich ist, weist ein ophthalmochirurgischer Injektor 1 einen Injektorkörper 2, eine Spitze 7, mittels welcher eine Intraokularlinse aus dem Injektor 1 herausdrückbar ist, einen ersten Stempel 3, der längsverschiebbar an dem Injektorkörper 2 gelagert ist und einen ersten Vorsprung 10 aufweist, eine Welle 5, die rotierbar in dem Injektorkörper 2 gelagert ist sowie eine erste Kulisse 9 und eine zweite Kulisse 11 aufweist, die jeweils um die Welle 5 gewunden sind, und einen zweiten Stempel 4 auf, der längsverschiebbar an dem Injektorkörper 2 gelagert ist und einen zweiten Vorsprung 12 aufweist. Der erste Vorsprung 10 greift in die erste Kulisse 9 ein, wodurch der erste Stempel 3 eingerichtet ist, durch eine Längsverschiebung bzw. Translation des ersten Stempels 3 eine Rotation der Welle 5 um eine Längsachse des Injektorkörpers 2 zu bewirken. Der zweite Vorsprung 12 greift in die zweite Kulisse 11 ein, wodurch die Welle 5 eingerichtet ist, durch die mittels des ersten Stempels 3 bewirkte Rotation der Welle 5 eine Längsverschiebung bzw. Translation des zweiten Stempels 4 bevorzugt entlang der Längsachse des Injektorkörpers 2 zu bewirken.

Damit die erste Kulisse 9 und die zweite Kulisse 11 gewunden sind und somit die Längsverschiebung des ersten Stempels 3 zu der Rotation der Welle 5 führt sowie die Rotation der Welle 5 zu der Längsverschiebung des zweiten Stempels führt, können die erste Kulisse 9 und die zweite Kulisse 11 Steigungen aufweisen, die von Null und von Unendlich verschieden sind. Die Steigung der ersten Kulisse 9 und die Steigung der zweiten Kulisse 11 können beispielsweise definiert sein als die Neigung der jeweiligen Kulisse 9, 11 gegen die Umfangsrichtung D der Welle 5. Hat die jeweilige Kulisse 9, 11 in einem Bereich eine Steigung von Unendlich, so würde diese einer Orientierung des Bereichs lediglich in der Axialrichtung z der Welle 5 entsprechen. Hat die jeweilige Kulisse 9, 11 in einem Bereich eine Steigung von Null, so würde dies einer Orientierung des Bereichs lediglich in der Umfangsrichtung D der Welle 5 entsprechen.

Figur 1 zeigt den Injektor 1 zu einem ersten Zeitpunkt, an dem der erste Stempel 3 so weit wie möglich von der Spitze 7 entfernt ist. Figur 3 zeigt den Injektor 1 zu einem dritten Zeitpunkt, an dem der erste Stempel 2 so nah wie möglich an der Spitze 7 angeordnet ist. Figur 2 zeigt den Injektor 1 zu einem zweiten Zeitpunkt, der zeitlich zwischen dem ersten Zeitpunkt und dem dritten Zeitpunkt liegt und an dem der erste Stempel 3 in einer Position zwischen der Position des ersten

Stempels 3 zum ersten Zeitpunkt und der Position des ersten Stempels 3 zum dritten Zeitpunkt angeordnet ist. Figuren 1 bis 3 zeigen, dass der Injektor 1 eingerichtet sein kann, die Längsverschiebung des ersten Stempels 3 hin zu der Spitze 7 in die Längsverschiebung des zweiten Stempels 4 hin zu der Spitze 7 zu übersetzen, wobei die Längsverschiebung des ersten Stempels 3 und des zweiten Stempels 4 im Wesentlichen in der Axialrichtung z der Welle 5 erfolgt. An der Form der ersten Kulisse 9 ist erkennbar, dass, wenn der erste Stempel 3 zu der Spitze 7 hin verschoben wird, die Welle 5 gesehen in einer Richtung von dem ersten Stempel 3 zu der Spitze 7 in die Rotation entgegen dem Uhrzeigersinn versetzt wird. An der Form der zweiten Kulisse 11 ist erkennbar, dass die Rotation der Welle 5 entgegen dem Uhrzeigersinn dazu führt, dass sich der zweite Stempel 4 hin zu der Spitze 7 verschiebt. Die Rotation entgegen dem Uhrzeigersinn ist ebenfalls an dem Zeiger 17 erkennbar, der an der der Spitze 7 abgewandten Stirnseite der Welle 5 eingezeichnet ist.

Um eine möglichst reibungsfreie Bewegung des ersten Vorsprungs 10 in der ersten Kulisse 9 und des zweiten Vorsprungs 12 in der zweiten Kulisse 11 zu ermöglichen, können die sich kontaktierenden Oberflächen der Kulissen 9, 11 und der Vorsprünge 10, 12 oberflächenbehandelt sein, der Injektor 1 kann einen Schmierstoff aufweisen, der eingerichtet ist, zwischen den sich kontaktierenden Oberflächen der Kulissen 9, 11 und der Vorsprünge 10, 12 zu wirken und/oder der Injektor 1 kann für jede der beiden Kulissen 9, 11 jeweils ein Kugellager aufweisen, wobei jeweils eine Kugel des Kugellagers in jedem der Vorsprünge 10, 12 eingearbeitet ist.

Der zweite Stempel 4 kann eingerichtet sein, durch seine Längsverschiebung die Intraokularlinse in die Spitze 7 zu drücken und anschließend aus dem Injektor 1 heraus zu drücken. Dazu kann an dem dem ersten Stempel 3 abgewandten Ende des zweiten Stempels 4 eine Stempelspitze 15 angeordnet sein, die von einem weichen Kissen oder einer Gabel gebildet ist. Die Längsverschiebung des zweiten Stempels 4 hin zu der Spitze 7 kann dazu führen, dass am Ende der Längsverschiebung des zweiten Stempels 4 der zweite Stempel 4 sich durch die vollständige Spitze 7 erstreckt. Auch ist es denkbar, dass am Ende der Längsverschiebung des zweiten Stempels 4 die Stempelspitze 15 außerhalb des Injektors 1 angeordnet ist.

Der Injektor 1 kann eine Lagerkammer aufweisen, in der die Intraokularlinse einzusetzen ist oder bereits eingesetzt ist. Zudem kann der Injektor 1 eine Faltkammer 6 aufweisen, in der bei der Längsverschiebung des zweiten Stempels 4 die Intraokularlinse gefaltet und komprimiert wird. Es ist denkbar, dass dazu die Faltkammer 6 sich in Richtung zu der Spitze 7 hin verjüngt. Es ist zudem denkbar, dass die Faltkammer 6 in der Axialrichtung z der Welle 5 zwischen der Lagerkammer und der Spitze 7 angeordnet ist. Die Spitze 7 kann sich in Richtung zu einem Ausgang der Spitze 7 hin verjüngen, wobei hier denkbar ist, dass die Spitze 7 sich weniger steil als die Faltkammer 6 verjüngt. Zudem ist denkbar, dass der Injektor 1 die Intraokularlinse aufweist. Dabei ist denkbar, dass die Intraokularlinse in dem Injektorkörper 2 und insbesondere in der Lagerkammer angeordnet ist. Aus Figuren 1 bis 3 ist außerdem ersichtlich, dass der erste Stempel 3 an seinem der Welle 5 abgewandten Längsende eine Daumenauflage 16 aufweisen kann.

Figuren 1 bis 3 zeigen, dass der Injektor 1 einen Anschlag 8 aufweisen kann, der eingerichtet ist, eine Bewegung der Welle 5 hin zu der Spitze 7 zu begrenzen. Zudem zeigen Figuren 1 bis 3, dass der Injektor 1 eine erste Verdrehsicherung 13 aufweisen kann, die eingerichtet ist, eine Rotation des ersten Stempels 3 relativ zu dem Injektorkörper 2 zu unterbinden. Dazu kann der erste Stempel 3 in einer Ebene, deren Normale parallel zu der Axialrichtung z der Welle 5 ist, einen von einem kreisförmigen Querschnitt verschiedenen Querschnitt haben, der formschlüssig in eine erste Öffnung des Injektorkörpers 2 eingreift. Zusätzlich oder alternativ kann der Injektor 1 eine zweite Verdrehsicherung 14 aufweisen, die eingerichtet ist, eine Rotation des zweiten Stempels 4 relativ zu dem Injektorkörper 2 zu unterbinden. Dazu kann der zweite Stempel 4 in einer Ebene, deren Normale parallel zu der Axialrichtung z der Welle 5 ist, einen von einem kreisförmigen Querschnitt verschiedenen Querschnitt haben, der formschlüssig in eine zweite Öffnung des Injektorkörpers 2 eingreift.

Wie es aus Figuren 1 bis 3 ersichtlich ist, kann die Welle 5 eine Hohlwelle sein und somit einen Hohlraum 18 aufweisen, wobei der erste Stempel 3 bei seiner Längsverschiebung zumindest teilweise und zumindest zeitweise in dem Hohlraum 18 angeordnet ist und/oder wobei der zweite Stempel 4 bei seiner Längsverschiebung zumindest teilweise und zumindest zeitweise in dem Hohlraum 18 angeordnet ist. Dabei können die erste Kulisse 9 und/oder die zweite Kulisse 11 sich in einer Radialrichtung der Welle 5 vollständig durch eine den Hohlraum 18 begrenzenden Wand der Welle 5 erstrecken. Alternativ ist denkbar, dass die erste Kulisse 9 als eine Nut ausgebildet ist und/oder dass die zweite Kulisse 11 als eine Nut ausgebildet ist.

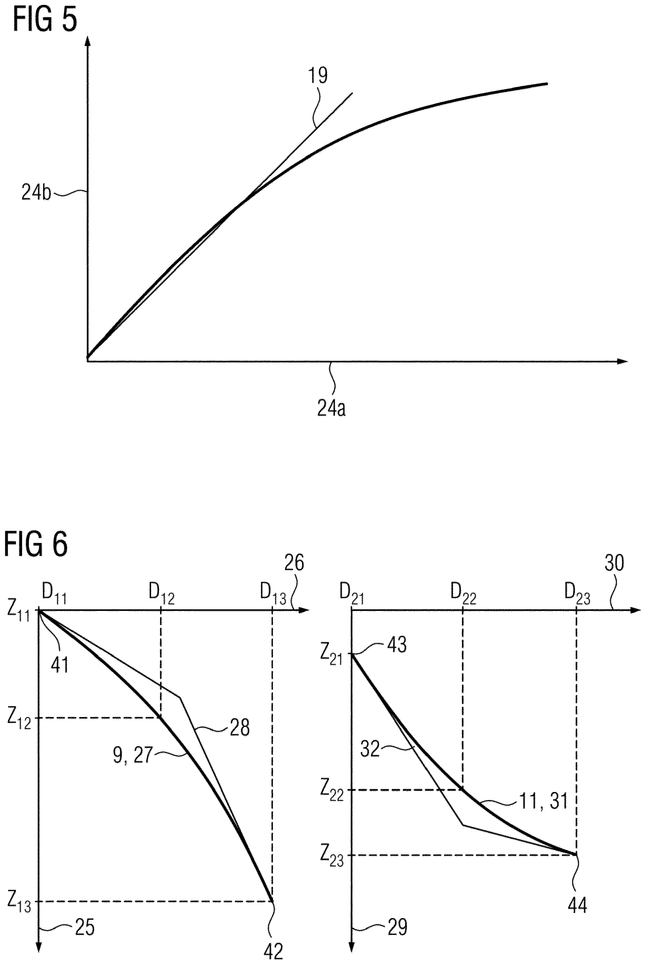

Figuren 1 bis 3 zeigen, dass die erste Kulisse 9 einen Anfangspunkt 41, an dem der erste Vorsprung 10 zu dem ersten Zeitpunkt angeordnet ist, und einen Endpunkt 42 aufweisen kann, an dem der erste Vorsprung 10 zu dem dritten Zeitpunkt angeordnet ist. Der Abstand von dem Anfangspunkt 41 der ersten Kulisse 9 zu dem Endpunkt 42 der ersten Kulisse 9 in der Axialrichtung z der Welle 5 entspricht dem maximalen Verschiebeweg des ersten Stempels 3. Zudem kann die zweite Kulisse 11 einen Anfangspunkt 43, an dem der zweite Vorsprung 12 zu dem ersten Zeitpunkt angeordnet ist, und einen Endpunkt 44 aufweisen, an dem der zweite Vorsprung 12 zu dem dritten Zeitpunkt angeordnet ist. Der Abstand von dem Anfangspunkt 43 der zweiten Kulisse 11 zu dem Endpunkt 44 der zweiten Kulisse 11 in der Axialrichtung z der Welle 5 entspricht dem maximalen Verschiebeweg des zweiten Stempels 4. Der Abstand des Anfangspunkts 41 der ersten Kulisse 9 zu dem Endpunkt 42 der ersten Kulisse 9 in der Umfangsrichtung D der Welle entspricht dem Abstand des ersten Vorsprungs 10 zu dem zweiten Vorsprung 12 in der Umfangsrichtung D der Welle 5 in einem radialen Abstand der Welle 5, auf dem die Kulissen 9, 11 angeordnet sind. Figuren 1 bis 3 zeigen, dass der maximale Verschiebeweg des ersten Stempels 3 länger als der maximale Verschiebeweg des zweiten Stempels 4 sein kann. Dies ist beispielhaft in Figur 5 in einer Auftragung dargestellt, in der über die horizontale Achse ein Verschiebeweg 24a des ersten Stempels 3 und über die vertikale Achse ein Verschiebeweg 24b des zweiten Stempels 4 aufgetragen ist. Ebenfalls eingetragen ist die Winkelhalbierende 19. Es ist zu erkennen, dass bei kurzen Verschiebewegen 24a des ersten Stempels 3 der zweite Stempel 4 einen längeren Verschiebeweg 24b als der Verschiebeweg 24a des ersten Stempels 3 zurücklegen kann und dass bei längeren Verschiebewegen 24a des ersten Stempels 3 der erste Stempel 3 einen längeren Verschiebeweg 24a als der Verschiebeweg 24b des zweiten Stempels 4 zurücklegen kann. Indem der erste Stempel 3 einen längeren Verschiebeweg 24a als der Verschiebeweg 24b des zweiten Stempels 4 zurücklegt, wird während der Längsverschiebung eine in der Axialrichtung z der Welle 5 auf den ersten Stempel 3 wirkende Kraft in eine stärkere, auf den zweiten Stempel 4 wirkende Kraft übersetzt.

In Figuren 6 bis 8 sind verschiedene Diagramme einer ersten beispielhaften Ausführungsform des Injektors 1 dargestellt. In Figur 6 sind in der linken Auftragung die Form der ersten Kulisse 9 und in der rechten Auftragung die Form der zweiten Kulisse 11 dargestellt. In der linken Auftragung ist über die horizontale Achse der Abstand 26 der ersten Kulisse 9 von dem Anfangspunkt 41 der ersten Kulisse 9 in der Umfangsrichtung D der Welle 5 und über die vertikale Achse der Abstand 25 der ersten Kulisse 9 von dem Anfangspunkt 41 der ersten Kulisse 9 in der Axialrichtung z der Welle 5 aufgetragen. In der rechten Auftragung ist über die horizontale Achse der Abstand 30 der zweiten Kulisse 11 von einem auf der Welle 5 liegenden Bezugspunkt in der Umfangsrichtung D der Welle 5 und über die vertikale Achse der Abstand 29 der zweiten Kulisse 11 von dem Bezugspunkt in der Axialrichtung z der Welle 5 aufgetragen. Die Auftragungen in Figur 6 entsprechen somit einer abgewickelten Oberfläche der Welle 5. Die Form der ersten Kulisse 9 kann, wie dargestellt, eine Beziér-Kurve 27 sein, wobei das dazugehörige Kontrollpolygon 28 eingezeichnet ist. Die Form der zweiten Kulisse 11 kann, wie dargestellt, eine Beziér-Kurve 31 sein, wobei das dazugehörige Kontrollpolygon 32 eingezeichnet ist. Anstelle der Beziér-Kurven 27, 31 sind auch andere Kurvenformen denkbar, wie beispielsweise Polynome oder Splines. In den Auftragungen sind (Du, zu) die Position des ersten Vorsprungs 10 zu dem ersten Zeitpunkt, (D₂₁, z₂₁) die Position des zweiten Vorsprungs 12 zu dem ersten Zeitpunkt, (D₁₂, z₁₂) die Position des ersten Vorsprungs 10 zu dem zweiten Zeitpunkt, (D₂₂, z₂₂) die Position des zweiten Vorsprungs 12 zu dem zweiten Zeitpunkt, (D₁₃, z₁₃) die Position des ersten Vorsprungs 10 zu dem dritten Zeitpunkt und (D₂₃, z₂₃) die Position des zweiten Vorsprungs 12 zu dem dritten Zeitpunkt. Weil der Abstand des ersten Vorsprungs 10 zu dem zweiten Vorsprung 12 in der Umfangsrichtung D der Welle bei dem Längsverschieben unveränderlich ist, gilt: D₁₃-D₁₁=D₂₃-D₂₁ und D₁₂-D₁₁=D₂₂-D₂₁.

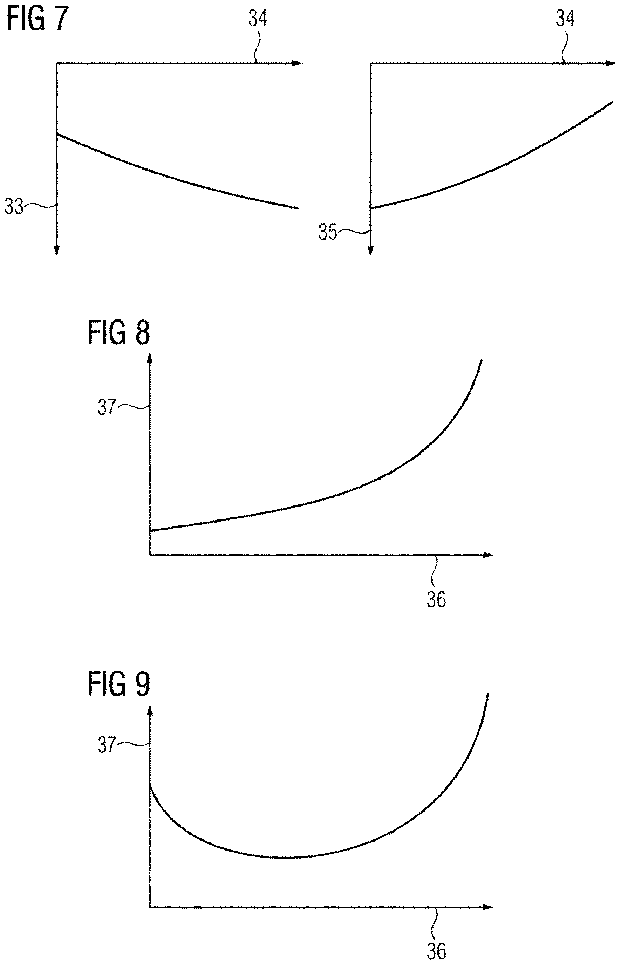

In Figur 7 sind in der linken Auftragung die Steigung 33 der ersten Kulisse 9 aus Figur 6 und in der rechten Auftragung die Steigung 35 der zweiten Kulisse 11 aus Figur 6 jeweils gegenüber einem Drehwinkel 34 der Welle 5 aufgetragen. In Figur 8 ist das Steigungsverhältnis 37 aus der Steigung 33 der ersten Kulisse 9 aus Figur 7 zu der Steigung 35 der zweiten Kulisse 11 aus Figur 7 gegen einen Verschiebeweg 36 des zweiten Stempels 4 dargestellt. Das Steigungsverhältnis 37 ist identisch zu einem Kraftverhältnis einer an dem zweiten Stempel 4 in einer Axialrichtung z der Welle 5 angreifenden Kraft zu einer an dem ersten Stempel 3 in einer Axialrichtung z der Welle 5 angreifenden Kraft. In Figur 8 ist das Steigungsverhältnis 37 gegen den Verschiebeweg 36 des zweiten Stempels 4 für eine zweite beispielhafte Ausführungsform des Injektors 1 aufgetragen, wobei bei der zweiten Ausführungsform die Formen der ersten Kulisse 9 und der zweiten Kulisse 11 verschieden von den Formen der ersten Kulisse 9 und der zweiten Kulisse 11 der ersten Ausführungsform sind.

Aus Figuren 8 und 9 ist ersichtlich, dass ein Steigungsverhältnis 37 aus der Steigung 33 der ersten Kulisse 9 an der Position, in der der erste Vorsprung 10 eingreift, zu der Steigung 35 der zweiten Kulisse 11 an der Position, in der der zweite Vorsprung 12 eingreift, bei der Längsverschiebung des ersten Stempels 3 und des zweiten Stempels 4 variieren kann. Es ist zudem erkennbar, dass das Steigungsverhältnis 37 zumindest abschnittsweise bei der Längsverschiebung des ersten Stempels 3 und des zweiten Stempels 4 hin zu der Spitze 7 größer werden kann. Bei beiden Ausführungsformen steigt das Steigungsverhältnis 37 entlang eines Endbereichs eines Verschiebewegs des zweiten Stempels 3 und damit auch entlang eines Endbereichs eines Verschiebewegs 36 des ersten Stempels 3 hin zu der Spitze 7 monoton an und insbesondere streng monoton an. Es ist denkbar, dass das Steigungsverhältnis 37 entlang des gesamten Verschiebewegs 36 des ersten Stempels 3 hin zu der Spitze 7 monoton ansteigt und insbesondere streng monoton ansteigt, wie es beispielsweise bei der ersten Ausführungsform der Fall ist. Alternativ ist denkbar, dass das Steigungsverhältnis 37 entlang eines Anfangsbereichs des Verschiebewegs des zweiten Stempels 4 und damit auch entlang eines Anfangsbereichs des Verschiebewegs 36 des ersten Stempels 3 hin zu der Spitze 7 monoton abfällt und insbesondere streng monoton abfällt, wie es bei der zweiten Ausführungsform der Fall ist.

Es ist insbesondere für die erste Ausführungsform und die zweite Ausführungsform denkbar, dass das Steigungsverhältnis 37 entlang eines Endbereichs eines Verschiebewegs des zweiten Stempels 4 und damit auch entlang eines Endbereichs eines Verschiebewegs 36 des ersten Stempels 4 hin zu der Spitze 7 größer als eins oder größer als 3,0 oder größer als 4,0 ist. Insbesondere für die zweite Ausführungsform ist denkbar, dass das Steigungsverhältnis 37 entlang eines Anfangsbereichs eines Verschiebewegs des zweiten Stempels 4 und damit auch entlang eines Anfangsbereichs eines Verschiebewegs 36 des ersten Stempels 3 hin zu der Spitze 7 größer als eins ist. Für die zweite Ausführungsform ist zudem denkbar, dass das Steigungsverhältnis 37 in einem Bereich des Verschiebeweges 36 des ersten Stempels 3, der zwischen dem Anfangsbereich und dem Endbereich des ersten Stempels 3 liegt, kleiner als 1 ist. Dadurch kann in Bereichen des Verschiebewegs 36 des ersten Stempels 3, in denen viel Kraft zum Verschieben der Intraokularlinse notwendig ist, die Übersetzung so gewählt werden, dass die an dem zweiten Stempel 4 in der Axialrichtung z wirkende Kraft stärker als die an dem ersten Stempel 3 in der Axialrichtung z der Welle 5 wirkende Kraft ist. Gleichzeitig kann in Bereichen des Verschiebewegs 36 des ersten Stempels 3, in denen nur wenig Kraft zum Verschieben der Intraokularlinse notwendig ist, die Übersetzung so gewählt werden, dass die an dem zweiten Stempel 4 in der Axialrichtung z der Welle 5 wirkende Kraft schwächer als die an dem ersten Stempel 3 in der Axialrichtung z der Welle 5 wirkende Kraft ist.

In Figur 4 ist ein Vergleich einer Kraft-Weg-Kurve 22 eines herkömmlichen Injektors, der keine Übersetzung aufweist, mit einer Kraft Weg-Kurve 23 des ersten Stempels 3 der zweiten Ausführungsform des Injektors 1 dargestellt. Aufgetragen ist über der horizontalen Achse der Verschiebeweg 20 des ersten Stempels 3 der zweiten Ausführungsform bzw. der Verschiebeweg 20 des Stempels des herkömmlichen Injektors und über die vertikale Achse die beim Verschieben aufgewendete Kraft 21. Es ist zu erkennen, dass der Verschiebeweg 20 der zweiten Ausführungsform wesentlich länger ist und dass der Anstieg der Kraft im Bereich 23d wesentlich geringer als der Anstieg der Kraft im Bereich 22d ist.

### Bezugszeichenliste

1 Injektor
2 Injektorkörper
3 erster Stempel
4 zweiter Stempel
5 Welle
6 Faltkammer
7 Spitze
8 Anschlag
9 erste Kulisse
10 erster Vorsprung
11 zweite Kulisse
12 zweiter Vorsprung
13 erste Verdrehsicherung
14 zweite Verdrehsicherung
15 Stempelspitze
16 Daumenauflage
17 Zeiger
18 Hohlraum
19 Winkelhalbierende
20 Verschiebeweg
21 Kraft
22 Kraft-Weg-Kurve eines Stempels eines herkömmlichen Injektors
23 Kraft-Weg-Kurve des ersten Stempels des erfindungsgemäßen Injektors
24a Verschiebeweg des ersten Stempels
24b Verschiebeweg des zweiten Stempels
25 Abstand der ersten Kulisse in Axialrichtung
26 Abstand der ersten Kulisse in Umfangsrichtung
27 erste Bézierkurve
28 erstes Kontrollpolygon
29 Abstand der zweiten Kulisse in Axialrichtung z
30 Abstand der zweiten Kulisse in Umfangsrichtung D
31 zweite Bézierkurve
32 zweites Kontrollpolygon
33 Steigung der ersten Kulisse
34 Drehwinkel der Welle
35 Steigung der zweiten Kulisse
36 Verschiebeweg des ersten Stempels
37 Steigungsverhältnis / Kraftverhältnis
41 Anfangspunkt der ersten Kulisse
42 Endpunkt der ersten Kulisse
43 Anfangspunkt der zweiten Kulisse
44 Endpunkt der zweiten Kulisse
z Axialrichtung
D Umfangsrichtung
z1x Axialposition des ersten Stempels zu einem x-ten Zeitpunkt D1x Umfangsposition des ersten Vorsprungs relativ zur Welle zu einem x-ten Zeitpunkt
z2x Axialposition des zweiten Stempels zu einem x-ten Zeitpunkt
D2x Umfangsposition des zweiten Vorsprungs relativ zur Welle zu einem x-ten Zeitpunkt

## Patentansprüche

1. Ophthalmochirurgischer Injektor mit einem Injektorkörper (2), einer Spitze (7), mittels welcher eine Intraokularlinse aus dem Injektor (1) herausdrückbar ist, einem ersten Stempel (3), der längsverschiebbar an dem Injektorkörper (2) gelagert ist und einen ersten Vorsprung (10) aufweist, einer Welle (5), die rotierbar in dem Injektorkörper (2) gelagert ist **dadurch gekennzeichnet, dass** die Welle eine erste Kulisse (9) und eine zweite Kulisse (11) aufweist, die jeweils um die Welle (5) gewunden sind, und einem zweiten Stempel (4), der längsverschiebbar an dem Injektorkörper (2) gelagert ist und einen zweiten Vorsprung (12) aufweist, wobei der erste Vorsprung (10) in die erste Kulisse (9) eingreift, wodurch der erste Stempel (3) eingerichtet ist, durch eine Längsverschiebung des ersten Stempels (3) eine Rotation der Welle (5) um eine Längsachse des Injektorkörpers (2) zu bewirken, und der zweite Vorsprung (12) in die zweite Kulisse (11) eingreift, wodurch die Welle (5) eingerichtet ist, durch die mittels des ersten Stempels (3) bewirkte Rotation eine Längsverschiebung des zweiten Stempels (4) zu bewirken.

2. Injektor gemäß Anspruch 1, wobei ein Steigungsverhältnis (37) aus der Steigung (33) der ersten Kulisse (9) an der Position, in der der erste Vorsprung (10) eingreift, zu der Steigung (35) der zweiten Kulisse (11) an der Position, in der der zweite Vorsprung (12) eingreift, bei der Längsverschiebung des ersten Stempels (3) und des zweiten Stempels (4) variiert.

3. Injektor gemäß Anspruch 2, wobei das Steigungsverhältnis (37) zumindest abschnittsweise bei der Längsverschiebung des ersten Stempels (3) und des zweiten Stempels (4) hin zu der Spitze (7) größer wird.

4. Injektor gemäß Anspruch 3, wobei das Steigungsverhältnis (37) entlang eines Endbereichs eines Verschiebewegs des zweiten Stempels (4) hin zu der Spitze (7) monoton ansteigt.

5. Injektor gemäß einem der Ansprüche 2 bis 4, wobei das Steigungsverhältnis (37) entlang eines Endbereichs eines Verschiebewegs des zweiten Stempels (4) hin zu der Spitze (7) größer als eins ist.

6. Injektor gemäß einem der Ansprüche 2 bis 5, wobei das Steigungsverhältnis (37) entlang eines Anfangsbereichs eines Verschiebewegs des zweiten Stempels (3) hin zu der Spitze (7) größer als eins ist.

7. Injektor gemäß einem der Ansprüche 1 bis 6, wobei die erste Kulisse (9) einen ersten Abschnitt und einen zweiten Abschnitt, die jeweils um die Welle (5) gewunden sind, sowie einen sich in einer Axialrichtung (z) der Welle (5) orientierten Abschnitt aufweist, via den der erste Vorsprung (10) durch seine Längsverschiebung weg von der Spitze (7) von dem ersten Abschnitt in den zweiten Abschnitt gelangen kann.

8. Injektor gemäß einem der Ansprüche 1 bis 7, wobei die Welle (5) eine Hohlwelle ist und somit einen Hohlraum (18) aufweist, wobei der erste Stempel (3) bei seiner Längsverschiebung zumindest teilweise und zumindest zeitweise in dem Hohlraum (18) angeordnet ist und/oder wobei der zweite Stempel (4) bei seiner Längsverschiebung zumindest teilweise und zumindest zeitweise in dem Hohlraum (18) angeordnet ist.

9. Injektor gemäß einem der Ansprüche 1 bis 8, wobei die Welle (5) eine Hohlwelle ist und somit einen Hohlraum (18) aufweist, wobei die erste Kulisse (9) und/oder die zweite Kulisse (11) sich in einer Radialrichtung der Welle (5) vollständig durch eine den Hohlraum (18) begrenzenden Wand der Welle (5) erstrecken.

10. Injektor gemäß einem der Ansprüche 1 bis 8, wobei die erste Kulisse (9) als eine Nut ausgebildet ist und/oder die zweite Kulisse (11) als eine Nut ausgebildet ist.

## Claims

1. Ophthalmosurgical injector having an injector body (2), a tip (7) by means of which an intraocular lens is able to be pushed out of the injector (1), a first plunger (3) which is mounted on the injector body (2) so as to be longitudinally displaceable and has a first protrusion (10), a shaft (5) which is mounted rotatably in the injector body (2), **characterized in that** the shaft has a first guide slot (9) and a second guide slot (11) which each wind around the shaft (5), and a second plunger (4) which is mounted on the injector body (2) so as to be longitudinally displaceable and has a second protrusion (12), wherein the first protrusion (10) engages in the first guide slot (9), with the result that the first plunger (3) is configured to bring about a rotation of the shaft (5) about a longitudinal axis of the injector body (2) as a result of a longitudinal displacement of the first plunger (3), and the second protrusion (12) engages in the second guide slot (11), with the result that the shaft (5) is configured to bring about a longitudinal displacement of the second plunger (4) as a result of the rotation brought about by means of the first plunger (3).

2. Injector according to Claim 1, wherein a pitch ratio (37) of the pitch (33) of the first guide slot (9) at the position in which the first protrusion (10) engages to the pitch (35) of the second guide slot (11) at the position in which the second protrusion (12) engages varies during the longitudinal displacement of the first plunger (3) and of the second plunger (4).

3. Injector according to Claim 2, wherein the pitch ratio (37) becomes greater at least in some portions during the longitudinal displacement of the first plunger (3) and of the second plunger (4) toward the tip (7).

4. Injector according to Claim 3, wherein the pitch ratio (37) increases monotonically along an end region of a displacement path of the second plunger (4) toward the tip (7).

5. Injector according to any of Claims 2 to 4, wherein the pitch ratio (37) is greater than one along an end region of a displacement path of the second plunger (4) toward the tip (7).

6. Injector according to any of Claims 2 to 5, wherein the pitch ratio (37) is greater than one along a starting region of a displacement path of the second plunger (3) toward the tip (7).

7. Injector according to any of Claims 1 to 6, wherein the first guide slot (9) has a first portion and a second portion which each wind around the shaft (5), and a portion oriented in an axial direction (z) of the shaft (5), via which portion the first protrusion (10) can pass from the first portion into the second portion as a result of its longitudinal displacement away from the tip (7).

8. Injector according to any of Claims 1 to 7, wherein the shaft (5) is a hollow shaft and thus has a cavity (18), wherein the first plunger (3) is arranged at least partially and at least temporarily in the cavity (18) during its longitudinal displacement and/or wherein the second plunger (4) is arranged at least partially and at least temporarily in the cavity (18) during its longitudinal displacement.

9. Injector according to any of Claims 1 to 8, wherein the shaft (5) is a hollow shaft and thus has a cavity (18), wherein the first guide slot (9) and/or the second guide slot (11) extend, in a radial direction of the shaft (5), all the way through a wall, bounding the cavity (18), of the shaft (5).

10. Injector according to any of Claims 1 to 8, wherein the first guide slot (9) is in the form of a groove and/or the second guide slot (11) is in the form of a groove.

## Revendications

1. Injecteur ophtalmochirurgical comprenant un corps d'injecteur (2), une pointe (7) au moyen de laquelle une lentille intraoculaire est apte à être expulsée de l'injecteur (1), un premier poinçon (3) qui est logé de manière à pouvoir coulisser longitudinalement sur le corps d'injecteur (2) et qui présente une première saillie (10), un arbre (5) étant monté rotatif dans le corps d'injecteur (2),
**caractérisé en ce que** l'arbre présente une première coulisse (9) et une deuxième coulisse (11) qui s'enroulent chacune autour de l'arbre (5), et un deuxième poinçon (4), qui est monté sur le corps d'injecteur (2) de manière à pouvoir être déplacé longitudinalement et qui présente une deuxième saillie (12), la première saillie (10) s'engageant dans la première coulisse (9), ce qui fait que le premier poinçon (3) est conçu pour provoquer une rotation de l'arbre (5) autour d'un axe longitudinal du corps d'injecteur (2) par un déplacement longitudinal du premier poinçon (3), et la deuxième saillie (12) s'engageant dans la deuxième coulisse, l'arbre (5) étant agencé de façon à provoquer un déplacement longitudinal du deuxième poinçon (4) par la rotation provoquée au moyen du premier poinçon (3).

2. Injecteur selon la revendication 1, dans lequel un rapport (37) de pas entre le pas de la première coulisse (9) jusqu'à la position dans laquelle la première saillie (10) est engagée et le pas (35) de la deuxième coulisse (11) jusqu'à la position dans laquelle la deuxième saillie (12) est engagée varie lors du déplacement longitudinal du premier poinçon (3) et du deuxième poinçon (4).

3. Injecteur selon la revendication 2, dans lequel le rapport de pas (37) augmente au moins par sections lors du déplacement longitudinal du premier poinçon (3) et du deuxième poinçon (4) vers la pointe (7).

4. Injecteur selon la revendication 3, dans lequel le rapport de pas (37) augmente de manière régulière le long d'une zone d'extrémité d'une course de déplacement du deuxième poinçon (4) vers la pointe (7).

5. Injecteur selon l'une des revendications 2 à 4, dans lequel le rapport de pas (37) le long d'une zone d'extrémité d'une course de déplacement du deuxième poinçon (4) vers la pointe (7) est supérieur à un.

6. Injecteur selon l'une des revendications 2 à 5, dans lequel le taux de pas (37) le long d'une partie initiale d'une course de déplacement du deuxième poinçon (3) vers la pointe (7) est supérieur à un.

7. Injecteur selon l'une des revendications 1 à 6, dans lequel la première coulisse (9) comprend une première portion et une deuxième portion enroulées respectivement autour de l'arbre (5), ainsi qu'une portion orientée selon une direction axiale (z) de l'arbre (5), via laquelle la première saillie (10) est apte à passer de la première portion à la deuxième portion par son déplacement longitudinal en écartement de la pointe (7).

8. Injecteur selon l'une des revendications 1 à 7, dans lequel l'arbre (5) est un arbre creux et présente donc une cavité (18), le premier poinçon (3) étant disposé au moins partiellement et au moins temporairement dans la cavité (18) lors de son déplacement longitudinal et/ou le deuxième poinçon (4) étant disposé au moins partiellement et au moins temporairement dans la cavité (18) lors de son déplacement longitudinal.

9. Injecteur selon l'une des revendications 1 à 8, dans lequel l'arbre (5) est un arbre creux et présente donc une cavité (18), la première coulisse (9) et/ou la deuxième coulisse (11) s'étendant, dans une direction radiale de l'arbre (5), entièrement à travers une paroi de l'arbre (5) délimitant la cavité (18).

10. Injecteur selon l'une des revendications 1 à 8, dans lequel la première coulisse (9) est réalisée sous la forme d'une rainure et/ou la deuxième coulisse (11) est réalisée sous la forme d'une rainure.
